# EUROPÄISCHE PATENTSCHRIFT

(11) **EP 4 648 717 B1**
(45) Veröffentlichungstag und Bekanntmachung des Hinweises auf die Patenterteilung: **25.11.2026**
(21) Anmeldenummer: 24721648.4
(22) Anmeldetag: 24.04.2024
(51) Int. Cl.: A61F 2/38, A61F 2/30

(54) **IMPLANTIERBARE PROTHESE ZUM ERSATZ DES MENSCHLICHEN KNIEGELENKS**
IMPLANTABLE PROSTHESIS FOR REPLACING THE HUMAN KNEE JOINT
PROTHÈSE IMPLANTABLE POUR LE REMPLACEMENT DE L'ARTICULATION DU GENOU HUMAIN

(30) Priorität: 25.04.2023 EP 23169657
(43) Veröffentlichungstag der Anmeldung: 19.11.2025
(73) Patentinhaber: Baumgart, Rainer, 80539 München (DE)
(72) Erfinder: Baumgart, Rainer, 80539 München (DE)
(74) Vertreter: Lambacher, Michael
(86) Internationale Anmeldenummer: PCT/EP2024/061203
(87) Internationale Veröffentlichungsnummer: WO 2024/223629

(56) Entgegenhaltungen:
- EP-A1- 3 838 228
- EP-A1- 4 122 427
- DE-A1- 2 334 265
- US-A1- 2009 299 482
- US-B2- 11 116 641

## Beschreibung

### GEBIET DER ERFINDUNG

Die Erfindung betrifft eine implantierbare Prothese zum Ersatz des menschlichen Kniegelenks, sowie gegebenenfalls auch der angrenzenden Knochenabschnitte.

### HINTERGRUND

Bösartige Knochentumore sind häufig kniegelenksnah am Femur oder an der Tibia lokalisiert, so dass nach Entfernung des betroffenen Knochenabschnittes sowohl ein Teil des Schaftes als auch der dazu gehörige femorale oder tibiale Gelenkanteil durch eine implantierbare Prothese ersetzt werden müssen.

Der entfernte Teil des Schaftes des betroffenen Knochens wird durch ein entsprechendes Knochenschaftersatzelement der Prothese ersetzt. Der Gelenkanteil des betroffenen Knochens wird durch den Gelenkanteil der Prothese ersetzt. In jedem Fall muss auch in den korrespondierenden Knochen ein Gelenkanteil implantiert werden, um eine Artikulation und eine Kopplung mit dem Gelenkanteil des betroffenen Knochens zu erreichen. Die Kopplung der Gelenkanteile erfolgt durch eine Gelenkeinrichtung, wie z.B. einen Scharnier- oder Kugelmechanismus, der eine Beweglichkeit zwischen Femur und Tibia ermöglichen soll, die der natürlichen Beweglichkeit des Kniegelenks möglichst nahekommt. Da natürliche Kniegelenke neben der Beugung auch eine geringfügige Drehung in der Transversalebene zulassen, wird es als vorteilhaft angesehen, wenn auch Kniegelenksprothesen diese Möglichkeit bieten. Die Verankerung der Prothese im Femur und der Tibia erfolgt jeweils durch eine in den Knochen eingebrachte Schaftverankerung. Falls eine spätere Entfernung vorgesehen ist, kann die Schaftverankerung mit glatter Oberfläche ausgeführt sein, so dass kein Knochen einwächst. Falls eine definitive Verankerung vorgesehen ist, kann die Oberfläche aufgeraut oder beschichtet sein, so dass Knochengewebe einwachsen kann. Alternativ kann die Schaftverankerung auch mit Knochenzement fixiert werden.

Da bösartige Knochentumore häufig bei Kindern im Wachstumsalter auftreten, kommt es im weiteren Verlauf nach der chirurgischen Entfernung des Tumors und der Implantation einer Tumorprothese durch den Wegfall der Wachstumsfuge auf der betroffenen Seite und durch das weitere Wachstum der Gegenseite zu einer Beinlängendifferenz. Das verzögerte Wachstum betrifft zwar in erster Linie den von dem Tumor selbst befallenen, aber auch den korrespondierenden Knochen, d.h. wenn der Tumor z.B. am Femur kniegelenksnah lokalisiert war, bleibt nicht nur das Femur sondern auch die Tibia im Wachstum zurück. Selbst wenn in diesem Fall die Schaftverankerung der Tibia glatt und poliert ausgeführt ist, so dass der Knochen grundsätzlich weiter wachsen kann, kommt es somit auch in dem korrespondierenden Knochen zu einer Wachstumsverzögerung, die zu der entstehenden Beinlängendifferenz beiträgt.

Zum Ersatz des menschlichen Kniegelenks und der angrenzenden Knochenabschnitte nach Entfernung von Knochentumoren bei Kindern im Wachstumsalter sind Prothesen bekannt, mit denen Beinlängendifferenzen ausgeglichen werden können. Konventionelle Prothesen werden entweder von außen mit einem Werkzeug aktiviert oder haben einen integrierten Motorantrieb, so dass sich das Schaftersatzelement teleskopartig verlängern kann. Derartige Prothesen sind z.B. in dem Dokument EP 2 468 216 B1 beschrieben. Wesentlich vorteilhafter sind Prothesen, bei denen nicht das Schaftersatzelement, sondern der verbliebene körpereigene Knochen verlängert wird.

Möglichkeiten, den nach Tumorentfernung verbliebenen Knochen trotz Tumorprothese zu verlängern, sind aus der EP 1 371 346 A1 und der EP 2 468 216 A1 bekannt. In der EP 1 371 346 A1 wird hierzu durch die Prothese in den verbliebenen Knochen ein Austausch der Schaftverankerung gegen einen Verlängerungsmarknagel offenbart, der nach einer Osteotomie die beiden Knochenfragmente langsam auseinanderzieht, so dass in dem sich vergrößernden Spalt neues Knochengewebe entstehen kann (Kallusdistraktionsmethode). Meist sind mehrere Verlängerungsschritte erforderlich, so dass der Verlängerungsmarknagel mehrfach getauscht werden muss. Nach Abschluss der Verlängerung muss der Verlängerungsmarknagel gegen eine dauerhaft einwachsende (beschichtete) Schaftverankerung ausgetauscht werden. Bei Lokalisation im proximalen Femur erfolgt der Austausch ebenfalls von proximal, bei Lokalisation im distalen Femur erfolgt der Austausch ebenfalls von distal, wobei das Gelenk der Prothese diesen Austausch nur bei vollständiger Entkopplung der Prothese ermöglicht, wodurch das Infektionsrisiko deutlich steigt. Die gleiche Problematik liegt bei Lokalisation des Tumors in der proximalen Tibia oder bei Verlängerungsbedarf der Tibia vor.

Die EP 2 468 216 A1 offenbart erstmals eine Lösung für den Austausch der Schaftverankerung am Femur in minimal invasiver Operationstechnik, indem der Wechsel der Komponenten von dem gegenüberliegenden Knochenende aus erfolgt. Sofern der Tumor am Femur lokalisiert ist und keine Längendifferenz am Unterschenkel besteht, können somit mit einer Prothese der in der EP 2 468 216 A1 beschriebenen Art wiederholte, große operative Zugänge über die Prothese vermieden und damit das Infektionsrisiko deutlich gesenkt werden.

Bei Verlängerungsbedarf der Tibia, sei es nach primärem Tumorbefall der Tibia oder wenn nach Tumorlokalisation am Femur auch die Tibia verkürzt ist und deshalb die vorläufige Schaftverankerung in der Tibia gegen einen Verlängerungsmarknagel und nach Wachstumsende der Verlängerungsmarknagel gegen eine beschichtete Schaftverankerung ausgetauscht werden soll, die in den Knochen dauerhaft einwächst, muss jedoch weiterhin jedes Mal die Prothese entkoppelt werden, wozu ein großer operativer Zugang mit einem erheblichen Infektionsrisiko erforderlich ist.

Der in der EP 2 468 216 A1 beschriebene Vorteil kommt somit letztendlich nur dann zum Tragen, wenn nicht gleichzeitig auch eine Unterschenkelverkürzung vorliegt, da die in der EP 2 468 216 A1 beschriebene Lösung am Unterschenkel nicht anwendbar ist. Für den häufigen Fall, dass die Verkürzung aber, so wie bereits erwähnt, auch den Unterschenkel betrifft, besteht nach der in EP 2 468 216 A1 beschriebenen Technik keine Möglichkeit, eine Verlängerung ohne Entkopplung der Prothese in minimal invasiver Technik durchzuführen. Somit steht den vorteilhaften Möglichkeiten eines Beinlängenangleichs an die Gegenseite durch Knochenwachstum weiterhin das erhebliche Infektionsrisiko durch wiederholte große operative Zugänge im Bereich der Prothese gegenüber.

Erstmals wird dieses Problem durch die in der EP 3 135 253 A1 offenbarte Prothese adressiert. Die dort beschriebene Prothese weist einen femoralen Gelenkanteil sowie einen tibialen Gelenkanteil auf, die über eine Gelenkeinrichtung gelenkig miteinander verbunden sind und die jeweils Durchgangsöffnungen aufweisen. Die Durchgangsöffnungen entsprechen einem Führungskanal, der je nach Kniebeugung einmal mit dem femoralen Gelenkanteil und einmal mit dem tibialen Gelenkanteil fluchtet und somit die Durchführung von Werkzeugen, eines Distraktionsmarknagels oder einer stabförmigen Schaftverankerung von außen sowohl in das Femur als auch in die Tibia ermöglicht, ohne dass die Prothese entkoppelt werden muss. Die EP 3 135 253 A1 offenbart somit zwar eine vorteilhafte Idee für die Durchführung von Werkzeugen, eines Distraktionsmarknagels oder einer stabförmigen Schaftverankerung, die dauerhafte Stabilität der Prothese wird jedoch durch die Durchgangsöffnungen beeinträchtigt, da diese direkt durch die in Gleiteingriff stehenden und für die Kraftübertragung vorgesehenen Gelenkkomponenten der Gelenkeinrichtung hindurchgehen.

Aus der DE 23 34 265 A1 ist eine Kniegelenkprothese mit ineinandergreifendem femoralen und tibialen Gelenkteil bekannt, wobei zwischen in Belastungsrichtung aufeinanderliegenden Gleitflächen Kunststofflagerteile vorgesehen sind.

Ferner sind aus der US 2009/299482 A1 und der US 11 116 641 B2 sind Kniegelenkprothesen bekannt, bei denen eine gelenkige Kopplung von tibialer und femoraler Gelenkkomponente über eine komplexe Kopplungseinrichtung im Gelenkzentrum erfolgt.

Ferner ist aus der EP 3 838 228 A1 eine Gelenkprothese, insbesondere eine Ellenbogengelenkprothese bekannt, bei der die beiden Gelenkkomponenten über sphärisch ausgebildete Gleitflächen beweglich miteinander gekoppelt sind; zur Begrenzung der Drehbewegung sind die Gleitflächen einer Gelenkkomponente mit Vorsprüngen, und die Gleitflächen der anderen Gelenkkomponente mit korrespondierenden Aussparungen versehen, in welche die Vorsprünge eingreifen.

Der hier vorliegenden Erfindung liegt die Aufgabe zugrunde, eine Prothese zum Ersatz des menschlichen Kniegelenks bereitzustellen, welche die Vorteile der in der EP 3 135 253 A1 beschriebenen Prothese aufweist und darüber hinaus den hohen biomechanischen Langzeitanforderungen und den funktionellen Ansprüchen an eine Kniegelenksendoprothese gerecht wird.

### KURZER ABRISS

Zur Lösung der oben genannten Aufgabe sowie weiterer Aufgaben wird eine implantierbare Prothese zum Ersatz eines menschlichen Kniegelenks gemäß Anspruch 1 bereitgestellt. Die Prothese umfasst eine Gelenkeinrichtung mit einem femoralen Gelenkkopf und einen mit dem Gelenkkopf zusammenwirkenden tibialen Gelenkfuß, die zur wahlweisen Realisierung wenigstens einer Gelenkbewegung in Form einer Beuge- und/oder Streckbewegung vorgesehen sind. Der Gelenkkopf der Prothese weist zwei erste Gleitflächen auf, die an gegenüberliegenden axialen Enden des Gelenkkopfs angeordnet sind und durch einen zentralen Gelenkkopfabschnitt räumlich voneinander getrennt sind. Der Gelenkfuß weist zwei zweite Gleitflächen auf, die an seitlich angeordneten Gelenkelementen des Gelenkfußes axial gegenüberliegend ausgebildet sind und mit den jeweiligen ersten Gleitflächen des Gelenkkopfs in Gleiteingriff stehen, wobei die zwei ersten Gleitflächen und die zwei zweiten Gleitflächen zur Realisierung wenigstens einer Beuge- und/oder Streckbewegung ausgebildet sind. Der zentrale Gelenkkopfabschnitt ist baulich für die Gelenkfunktion entbehrlich und weist zwei Führungskanäle auf, die jeweils zur Durchführung eines Werkzeuges, eines Distraktionsmarknagels oder einer femoralen oder tibialen Schaftverankerung vorgesehen sind.

Der Gelenkfuß ist mit dem Gelenkkopf über die axial nach außen verlagerten ersten Gleitflächen und zweiten Gleitflächen gelenkig miteinander gekoppelt. Somit werden durch die erfindungsgemäße Gelenkeinrichtung die zur gelenkigen Kopplung beitragenden Gelenkkomponenten (Gleitflächen) vollständig von dem zentralen Gelenkkopfabschnitt und dem darin realisierten wenigstens einen Führungskanal entkoppelt. Dadurch wird eine dauerhaft stabile Prothese realisiert, welche den biomechanischen Langzeitanforderungen und den funktionellen Ansprüchen an eine Kniegelenksendoprothese gerecht wird, und gleichzeitig auch die in der EP 3 135 253 A1 beschriebene vorteilhafte Durchführung von Werkzeugen, eines Distraktionsmarknagels oder einer stabförmigen Schaftverankerung ermöglicht.

Die Prothese kann ferner ein femorales Knochenschaftersatzelement und/oder ein tibiales Knochenschaftersatzelement zum Ersatz eines entfernten Knochens aufweisen. Zum Ersatz des entfernten femoralen Gelenks kann der Gelenkkopf mit dem distalen Ende des femoralen Knochenschaftersatzelements verbunden sein oder eine Einheit bilden. Zum Ersatz des entfernten tibialen Gelenks kann der Gelenkfuß mit dem proximalen Ende des tibialen Knochenschaftersatzelements verbunden sein oder eine Einheit bilden.

Der Gelenkkopf kann zwei äußere Gelenkkopfabschnitte aufweisen, die in axialer Richtung durch den zentralen Gelenkkopfabschnitt räumlich voneinander getrennt sind; am axialen äußeren Ende eines jeden äußeren Gelenkkopfabschnittes kann eine der zwei ersten Gleitflächen ausgebildet sein. Dadurch werden die für die bewegliche Kopplung vorgesehenen Gleitflächen (Gelenkflächen) axial möglichst weit nach außen gelegt, wodurch selbst bei Gelenkeinrichtungen kleiner Größe/Dimensionierung eine stabile gelenkige Kopplung erreicht wird.

Die Größe/Dimensionierung der Prothese hängt insbesondere im Gelenkbereich von den anatomischen Gegebenheiten ab. Um eine möglichst einfache und kompakte Gelenkeinrichtung zu realisieren, kann der Gelenkkopf der Prothese im Wesentlichen walzenförmig (zylinderförmig) oder hohlzylinderförmig ausgebildet sein. Der walzenförmige oder hohlzylinderförmige Gelenkkopf kann eine vorgegebene axiale Ausdehnung (also Ausdehnung entlang seiner Rotationsachse) und eine vorgegebene radiale Ausdehnung (Ausdehnung senkrecht zu seiner Rotationsachse) aufweisen, wobei die Ausdehnung (Abmessung) in axialer und radialer Richtung durch die umgebenden Weichteile limitiert bzw. vorgegeben wird. Entsprechend sind die zwei ersten Gleitflächen an oder in der Nähe der gegenüberliegenden axialen äußeren Endabschnitte des walzenförmigen oder hohlzylinderförmigen Gelenkkopfs ausgebildet; der zentrale Gelenkkopfabschnitt ist mittig zwischen den zwei axialen Endabschnitten des walzenförmigen oder hohlzylinderförmigen Gelenkkopfs ausgebildet.

Der mit dem Gelenkkopf gelenkig gekoppelte Gelenkfuß kann zur beweglichen Aufnahme des (walzenförmigen oder hohlzylinderförmigen) Gelenkkopfs ausgebildet sein. Insbesondere kann der Gelenkfuß gabelförmig ausgebildet sein mit zwei seitlich (medial und lateral) angeordneten Gelenkelementen, die axial voneinander beabstandet angeordnet sind. Beispielsweise können die axial voneinander beabstandeten Gelenkelemente seitlich außen liegend angeordnet sein. Sie können jeweils von medial oder lateral (formschlüssig) mit dem Gelenkfuß verbunden werden. Ferner können die seitlichen Gelenkelemente im Vergleich zu den äußeren Kopfabschnitten bzw. axialen Enden des Gelenkkopfs axial weiter außen angeordnet sein; dadurch kann der Gelenkkopf in dem zwischen den zwei seitlich angeordneten Gelenkelementen entstandenen Gelenkraum platzsparend positioniert werden.

Der Gelenkfuß kann ein zentrales Gelenkelement aufweisen, wobei die seitlichen Gelenkelemente am zentralen Gelenkelement lösbar befestigbar sind, wobei optional die zwei seitlichen Gelenkelemente Justiernasen aufweisen, die dazu ausgebildet sind, in entsprechende Justiernuten am zentralen Gelenkelement formschlüssig einzugreifen.

Ferner können die zwei zweiten Gleitflächen des Gelenkfußes an den Innenseiten der zwei seitlich angeordneten Gelenkelemente angeordnet sein, so dass jeweils eine zweite Gleitfläche mit einer entsprechenden ersten Gleitfläche an den beiden gegenüberliegenden axialen Enden des Gelenkkopfs in Gleiteingriff kommen kann. Mit anderen Worten sind der Gelenkkopf und der Gelenkfuß über ihre jeweiligen axial außenliegenden Gleitflächen drehbar (gelenkig) miteinander gekoppelt. Diese gabelförmige Außenlagerung von Gelenkkopf und Gelenkfuß erhöht die Stabilität der Gelenkeinrichtung. Gleichzeitig weist die Gelenkeinrichtung im Zentrum keine zur gelenkigen Kopplung beitragenden Gelenkkomponenten auf. Denn der zentrale Gelenkkopfabschnitt trägt zur gelenkigen Kopplung nicht bei; ihm kommt keine Gelenkfunktion, insbesondere keine kraftübertragende Funktion zu. Vielmehr kommt dem zentralen Gelenkkopfabschnitt eine Führungsfunktion zur Einführung eines Werkzeugs, eines Distraktionsmarknagels und/oder einer femoralen oder tibialen Schaftverankerung zu. Im Gegensatz zu der in der EP 3 135 253 B1 beschriebenen Gelenkeinrichtung sind mit der erfindungsgemäßen Gelenkeinrichtung die Gelenkfunktion und der damit einhergehende Kraftfluss von der Führungsfunktion für die Verankerungselemente räumlich und funktionell vollständig entkoppelt.

### Gelenkachse - Bewegungsumfang der Gelenkeinrichtung

Die Gelenkeinrichtung kann ausgehend von einer Streckstellung dazu ausgebildet sein, eine Beugebewegung von bis zu 120° zu realisieren. Die Beugebewegung ist eine Drehbewegung um eine virtuelle Gelenkachse, die durch die oben beschriebene bewegliche Lagerung von Gelenkkopf und Gelenkfuß definiert wird. Entsprechend ist mit "axial" oder "axialer Richtung" eine Richtung parallel zur virtuellen Gelenkachse und mit "radial oder radialer Richtung" eine Richtung senkrecht zur Gelenkachse gemeint.

Zur Begrenzung der Drehbewegung um die Gelenkachse, insbesondere der Streckbewegung, kann die Gelenkeinrichtung einen Streckanschlag aufweisen. Der Streckanschlag kann durch wenigstens eine nach ventral hin (also zur Vorderseite hin) ausgebildete Anschlagfläche am Gelenkkopf realisiert sein. Die wenigstens eine Anschlagfläche am Gelenkkopf kann dadurch realisiert werden, dass der walzenförmige oder hohlzylinderförmige Gelenkkopf in radialer Richtung eine Körperausdehnung aufweist, die zur Ventralseite des Gelenkkopfs hin lokal (leicht) zunimmt. Mit anderen Worten kann der Gelenkkopf an seiner Außenseite in Umfangsrichtung nicht vollständig kreisrund ausgebildet sein, sondern eine vom kreisrunden Verlauf abweichende, und zur Ventralseite hin gerichtete lokale Materialzunahme aufweisen. Durch diese ventralseitige, lokale Materialzunahme (Ausdehnungszunahme) des Gelenkkopfs wird eine Anschlagfläche geschaffen, die mit einem entsprechenden Anschlagelement des Gelenkfußes zusammenwirken kann und somit eine Überstreckung der Prothese verhindert.

Um den Effekt des hier beschriebenen ventralen Streckanschlags noch zu vergrößern, kann die virtuelle Gelenkachse in Relation zur Mitte der Femurlängsachse (geringfügig) in dorsaler Richtung verlagert sein.

Die Gelenkeinrichtung kann ferner ausgebildet sein, um zusätzlich zu der hier beschriebenen Beugebewegung und/oder Streckbewegung noch eine eingeschränkte (begrenzte) Rotationsbewegung um die Tibiaschaftachse zu realisieren. Insbesondere kann die Gelenkeinrichtung dazu ausgebildet sein, die zusätzliche begrenzte Rotationsbewegung nur dann zu ermöglichen, wenn die Gelenkeinrichtung sich in einer Beugestellung (und somit in der Funktionsstellung einer Kniebeugung) befindet, was den physiologischen Gegebenheiten entspricht. In der Streckstellung wird hingegen eine Rotationsbewegung um die Tibiaschaftachse durch das Anschlagselement blockiert.

### Ausbildung der Gleitflächen (Gelenkflächen)

Der Bewegungsumfang der Gelenkeinrichtung, wie oben beschrieben, kann durch entsprechende Ausgestaltung der zwei ersten Gleitflächen (Gelenkflächen) und der mit den zwei ersten Gleitflächen korrespondierenden zwei zweiten Gleitflächen (Gelenkflächen) realisiert werden.

Gemäß einer ersten Ausgestaltungsvariante können die zwei ersten Gleitflächen und die zwei zweiten Gleitflächen Abschnitte einer Zylindermantelfläche sein, deren Radien (Durchmesser) aufeinander abgestimmt sind, so dass eine spielfreie Gleitbewegung und damit eine Drehung um die Gelenkachse möglich ist. Mit anderen Worten können die zwei ersten Gleitflächen jeweils radial gekrümmte Gleitflächen sein, die in Umfangsrichtung jeweils einen ersten radialen Abstand (erster Radius) von der Gelenkachse (Rotationsachse des Gelenkkopfs) aufweisen. In einer Schnittbetrachtung senkrecht zur Rotationsachse können die ersten Gleitflächen jeweils kreisförmig ausgebildet sein. Ebenso können die zwei zweiten Gleitflächen jeweils radial gekrümmte Gleitflächen sein, die in Umfangsrichtung jeweils einen zweiten radialen Abstand (zweiter Radius) von der Gelenkachse (Rotationsachse des Gelenkkopfs) aufweisen. In einer Schnittbetrachtung senkrecht zur Rotationsachse können die zweiten Gleitflächen jeweils kreisförmig ausgebildet sein. Der zweite Radius der zweiten Gleitflächen kann auf den ersten Radius der ersten Gleitflächen derart abgestimmt sein, dass an medialer und lateraler Seite der Gelenkeinrichtung jeweils eine erste Gleitfläche mit einer zweiten Gleitfläche in Gleiteingriff kommt, und somit eine spielfreie Drehbewegung um die Gelenkachse ermöglicht.

Beispielsweise können die jeweiligen ersten Gleitflächen die Innenfläche eines jeweiligen Außenringes sein, der an den gegenüberliegenden äußeren Gelenkkopfabschnitten angeordnet ist und somit an den jeweiligen axialen Enden des Gelenkkopfs axial nach außen vorsteht. Ferner können die jeweiligen zweiten Gleitflächen die Außenfläche eines jeweiligen Innenringes sein, der an den jeweiligen gegenüberliegenden seitlichen Gelenkelementen axial nach innen vorsteht. Alternativ können die jeweiligen ersten Gleitflächen die Außenfläche eines jeweiligen Innenringes sein, der an den gegenüberliegenden äußeren Gelenkkopfabschnitten angeordnet ist und somit an den jeweiligen gegenüberliegenden axialen Enden des Gelenkkopfs axial nach außen vorsteht. Ferner können die jeweiligen zweiten Gleitflächen Innenflächen eines jeweiligen Außenringes sein, der an den jeweiligen gegenüberliegenden seitlichen Gelenkelementen axial nach innen vorsteht. Im montierten Zustand der Gelenkeinrichtung greifen an beiden axialen Enden die jeweiligen Innenringe in die jeweiligen Außenringe ein, wodurch die ersten Gleitflächen jeweils mit den zweiten Gleitflächen gleitend in Kontakt kommen. Durch die hier beschriebene kreisförmig gekrümmte Ausbildung der ersten Gleitflächen und zweiten Gleitflächen relativ zur Gelenkachse kann eine Drehbewegung um die Gelenkachse der Gelenkeinrichtung und somit die oben beschriebene wahlweise Beuge- und/oder Streckbewegung realisiert werden.

Um der physiologischen Kniegelenksfunktion möglichst nahe zu kommen, können gemäß einer zweiten Ausgestaltungsvariante die zwei ersten Gleitflächen und die zwei zweiten Gleitflächen auch jeweils radial gekrümmte Abschnittsflächen eines Kugelmantels mit dem virtuellen Kugelzentrum in Zentrum der Kniegelenksprothese sein, so dass neben der Beugebewegung auch eine umschriebene Drehbewegung um die Bein-Längsachse möglich ist.

Mit anderen Worten können in der zweiten Ausgestaltungsvariante die ersten Gleitflächen und die zweiten Gleitflächen jeweils sphärisch oder zumindest teilweise sphärisch ausgebildet sein. Gemäß einer Variante können die zwei ersten Gleitflächen jeweils (teilweise) sphärisch konvex und die korrespondierenden zweiten Gleitflächen jeweils (teilweise) sphärisch konkav ausgebildet sein und somit jeweils ein Teilsegment einer Kugeloberfläche bilden. Beispielsweise können die (teilweise) sphärisch konvex ausgebildeten ersten Gleitflächen an den gegenüberliegenden äußeren Gelenkkopfabschnitten angeordnet sein und somit an den jeweiligen axialen Enden des Gelenkkopfs axial nach außen vorstehen; die (teilweise) sphärisch konkav ausgebildeten zweiten Gleitflächen können hingegen an den Innenseiten der axial gegenüberliegenden seitlichen Gelenkelelemente des Gelenkfußes eingelassen sein. Gemäß einer alternativen Variante können die zwei ersten Gleitflächen jeweils (teilweise) sphärisch konkav und die korrespondierenden zweiten Gleitflächen jeweils (teilweise) sphärisch konvex ausgebildet sein. In diesem Fall können die (teilweise) sphärisch konkav ausgebildeten ersten Gleitflächen an den gegenüberliegenden axialen Enden des Gelenkkopfs eingelassen sein, während die (teilweise) sphärisch konvex ausgebildeten zweiten Gleitflächen an den Innenseiten der axial gegenüberliegenden seitlichen Gelenkelemente des Gelenkfußes hervorstehen.

Die (teilweise) sphärisch konvex ausgebildeten Gleitflächen können die Oberflächen entsprechender Vorsprünge sein, die entweder an den Innenseiten der seitlichen Gelenkelemente des Gelenkfußes oder an den axialen Enden (axialen Außenseiten) des Gelenkkopfs ausgebildet sind. Ferner können die (teilweise) sphärisch konkav ausgebildeten Flächen die Oberflächen entsprechender Nuten sein, die entweder an den Innenseiten der seitlichen Gelenkelelemente des Gelenkfußes oder an den axialen Enden (axialen Außenseiten) des Gelenkkopfs ausgebildet sind. Im montierten Zustand der Gelenkeinrichtung greifen an beiden axialen Enden die jeweiligen (teilweise) konvex ausgebildeten sphärischen Gleitflächen in die entsprechenden, jeweils (teilweise) konkav ausgebildeten sphärischen Gleitflächen ein, wodurch zwischen dem Gelenkkopf und dem Gelenkfuß eine (partielle) Kugellagerung realisiert wird, welche nicht nur eine Drehbewegung um die Gelenkachse zur Realisierung einer Beuge- und Streckbewegung, sondern zusätzlich noch eine begrenzte Drehbewegung (Rotationsbewegung um einige Grad) um die Bein-Längsachse ermöglicht. Dadurch kann die natürliche Beweglichkeit eines Kniegelenkes noch besser nachgebildet werden.

In beiden Ausgestaltungsvarianten kann das virtuelle Drehzentrum des Zylinders oder der Kugel etwas nach dorsal verlagert sein; dadurch kann zusätzlich noch der oben beschriebene Streckanschlag in vorteilhafter Weise verbessert werden. Wenn die Gleitflächen einem Kugelmantelabschnitt entsprechen, kann der Streckanschlag ferner verhindern, dass in voller Streckstellung der Gelenkfuß senkrecht zur Gelenkachse zusätzlich rotierbar ist. Mit anderen Worten ist die (begrenzte) Rotation des Gelenkfußes um die Tibiaachse und somit relativ zum Gelenkkopf um eine Achse senkrecht zur Gelenkachse nur dann möglich, wenn die Gelenkeinrichtung eine Beugestellung (und somit die Funktionsstellung einer Kniebeugung) einnimmt, was den physiologischen Gegebenheiten entspricht.

Unabhängig von der hier beschriebenen konkreten Ausgestaltungsvarianten können die zwei ersten Gleitflächen und/oder die zwei zweiten Gleitflächen jeweils eine biokompatible Beschichtung zur Reibungsverminderung aufweisen. Alternativ können die zwei ersten Gleitflächen und/oder zwei zweiten Gleitflächen jeweils aus einer zusätzlichen Materialkomponente gefertigt sein, welche verschleißresistent ist und zur Reibungsverminderung zwischen dem Gelenkkopf und dem Gelenkfuß beiträgt. Denkbar ist der Einsatz jeglicher Art von Materialpaarung bzw. Oberflächenbeschichtung für die ersten und zweiten Gleitflächen, wie sie aus dem Stand der Technik bekannt sind.

### Ausbildung der Führungskanäle

Wie oben beschrieben, sind zwei Führungskanäle in dem zentralen Gelenkkopfabschnitt ausgebildet. Jeder Führungskanal kann als Durchgangsbohrung im zentralen Gelenkkopfabschnitt ausgebildet sein und derart dimensioniert sein, dass ein Werkzeug, ein Distraktionsmarknagel und/oder eine femorale oder tibiale Schaftverankerung durch den Führungskanal hindurchführbar ist/sind. Insbesondere kann jeweils ein Führungskanal derart im zentralen Gelenkkopfabschnitt angeordnet (bzw. ausgerichtet) sein, dass er mit einer Durchgangsöffnung eines angrenzenden femoralen Knochenschaftersatzelements oder mit einer Durchgangsöffnung des Gelenkfußes in Flucht kommt, wenn die Gelenkeinrichtung einen vorgegebenen Beugungswinkel realisiert. Der vorgegebene Beugungswinkel kann hierbei einem Winkel entsprechen, welchen der Gelenkkopf und der Gelenkfuß der Gelenkeinrichtung bei der Realisierung einer einer Kniebeugung entsprechenden Funktionsstellung einnehmen.

Ähnlich wie in der EP 3 135 253 A1 kann der vorgegebene Beugungswinkel, bei dem jeweils ein Führungskanal mit einer Durchgangsöffnung eines angrenzenden femoralen Knochenschaftersatzteils oder dem tibialen Gelenkanteil (Gelenkfußes) fluchtet, im Bereich von 20° bis 90°, vorzugsweise im Bereich von 30° bis 60°, besonders bevorzugt im Bereich von 30° bis 45° liegen. Für die angegebenen Beugungswinkelbereiche wird wiederum die Streckstellung als Referenz genommen. Durch die hier beschriebene Ausbildung kann auf einfache und schonende Weise ein Werkzeug, ein Distraktionsmarknagel oder eine femorale oder tibiale Schaftverankerung über den jeweiligen Führungskanal in die angrenzende Durchgangsöffnung des femoralen und/oder tibialen Gelenkelements bzw. des Knochenschaftersatzelements und weiter in den jeweiligen Knochen eingeführt werden, ohne dass die

Gelenkeinrichtung der Prothese entkoppelt werden muss. Dadurch kann der operative Eingriff (beispielsweise beim Ersatz eines Distraktionsmarknagels durch eine tibiale Schaftverankerung) erheblich reduziert und das Infektionsrisiko deutlich gesenkt werden.

Dem zentralen Gelenkkopfabschnitt und somit dem zentralen Teil der Kniegelenksprothese kommt somit allein eine Führungsfunktion zur Einführung eines Werkzeugs, eines Distraktionsmarknagels und/oder einer femoralen oder tibialen Schaftverankerung zu. Im Gegensatz zu der in der EP 3 135 253 B1 beschriebenen Gelenkeinrichtung sind mit der erfindungsgemäßen Gelenkeinrichtung die Gelenkfunktion und die Führungsfunktion für die Verankerungselemente räumlich und funktionell vollständig entkoppelt. Durch die vorteilhafte Anordnung der Gelenkelemente möglichst weit außen ist zudem eine dauerhafte Stabilität gewährleistet.

Der femorale Gelenkanteil (Gelenkkopf) kann ventralseitig ferner eine Gleitfläche für eine Kniescheibe aufweisen.

### KURZE BESCHREIBUNG DER FIGUREN

Weitere Vorteile und Aspekte der Erfindung werden anhand von Zeichnungen erläutert. Es zeigen:
- Figur 1: eine Außenansicht einer erfindungsgemäßen Prothese zum Ersatz des menschlichen Kniegelenks in leichter Beugestellung;
- Figur 2: eine Explosionsdarstellung der in der Figur 1 dargestellten Prothese;
- Figuren 3a, 3b: weitere Ansichten der in der Figur 1 dargestellten Prothese;
- Figur 4: eine Explosionsdarstellung einer weiteren erfindungsgemäßen Prothese zum Ersatz des menschlichen Kniegelenks; und
- Figuren 5a, 5b: weitere Ansichten der in der Figur 4 dargestellten Prothese.

### DETAILLIERTE BESCHREIBUNG

Aspekte der vorliegenden Erfindung werden anhand exemplarischer Ausführungsformen weiter beschrieben.

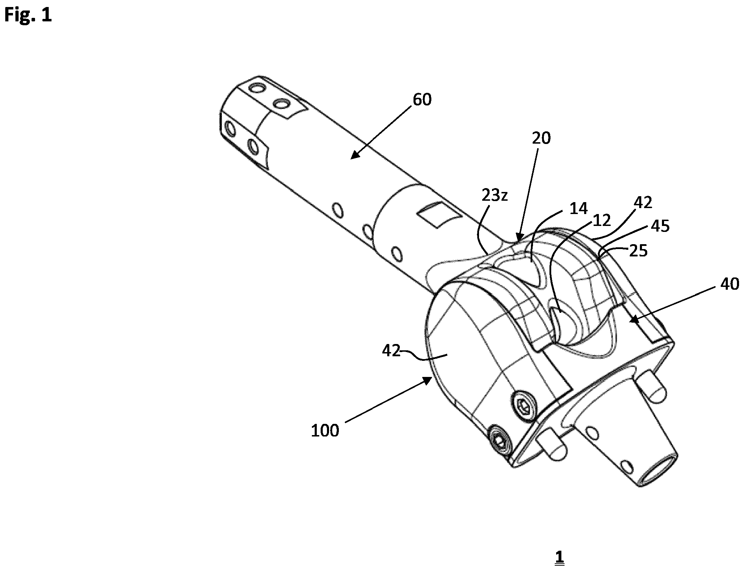

Figur 1 zeigt eine Außenansicht einer erfindungsgemäßen Prothese 1 in leichter Beugestellung.

Die Prothese 1 umfasst eine Gelenkeinrichtung 100 mit einem Gelenkkopf 20 und einem mit dem Gelenkkopf 20 gelenkig zusammenwirkenden Gelenkfuß 40. In der Figur 1 wird die Prothese 1 mit einem femoralen Knochenschaftersatzelement 60 gezeigt, das mit seinem distalen Ende mit dem Gelenkkopf 20 verbunden ist oder auch eine Einheit bilden kann. Ferner kann der Gelenkfuß 40 der Prothese 1 mit dem proximalen Ende eines tibialen Knochenschaftersatzelements (nicht gezeigt) verbunden sein oder auch eine Einheit bilden. Je nach Einsatz der Prothese 1 kann das femorale bzw. tibiale Knochenschaftersatzelement auch entfallen.

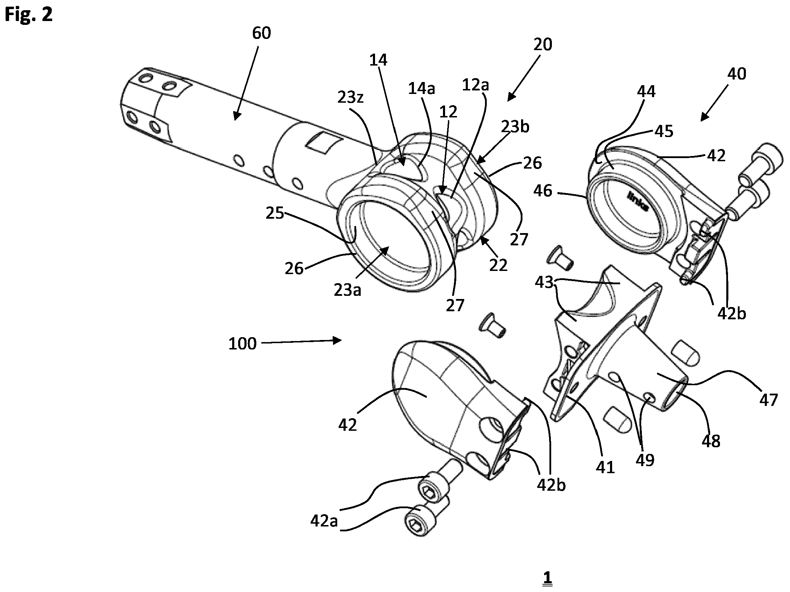

In Zusammenhang mit der Figur 2 wird die die Gelenkeinrichtung 100 der Prothese 1 weiter beschrieben.

Der Gelenkkopf 20 der Gelenkeinrichtung 100 ist walzenförmig (zylinderförmig) ausgebildet. Der Walzenkörper 22 des Gelenkkopfs 20 definiert eine Rotationsachse, welche der Gelenkachse entspricht. Ferner weist der Walzenkörper 22 einen mittig angeordneten Körperabschnitt (im Folgenden als zentraler Gelenkkopfabschnitt 23z bezeichnet) sowie zwei äußere Körperabschnitte (im Folgenden als äußere Gelenkkopfabschnitte 23a/23b bezeichnet) auf, die durch den zentraler Gelenkkopfabschnitt 23z in axialer Richtung räumlich voneinander getrennt werden. Der Gelenkkopf 20 kann über seinen zentralen Gelenkkopfabschnitt 23z mit dem femoralen Knochenschaftersatzelement 60 gekoppelt sein oder mit diesem eine Einheit bilden oder direkt über eine Schaftverankerung mit dem Femur verbunden sein.

Das femorale Knochenschaftersatzelement 60 kann ferner eine Durchgangsöffnung aufweisen (in Figur 2 nicht sichtbar, da verdeckt). Diese Durchgangsöffnung kann in einen Führungskanal 12 des zentralen Gelenkkopfabschnittes 23z münden.

Im Bereich der zwei äußeren Gelenkkopfabschnitte 23a, 23b weist der Gelenkkopf 20 jeweils einen ringförmigen Vorsprung 26 auf. Die beiden ringförmigen Vorsprünge 26 bilden jeweils einen Außenring 26 an den gegenüberliegenden axialen Enden des Gelenkkopfs (bzw. seines Walzenkörpers 22). Die Innenfläche 25 des jeweiligen Außenrings 26 bildet eine erste kreisförmige Gleitfläche 25 (Gelenkfläche 25) der Gelenkeinrichtung 100.

Ferner weist der Gelenkkopf 20 an seiner radialen Außenfläche wenigstens eine Anschlagfläche 27 auf. Die Anschlagfläche 27 ist an beiden äußeren Gelenkkopfabschnitten 23a, 23b ausgebildet. Konstruktionstechnisch kann die Anschlagfläche 27 dadurch realisiert werden, dass der walzenförmige oder hohlzylinderförmige Gelenkkopf 20 an der Außenseite eine von einer kreis- oder walzenförmigen Körperform lokal abweichende Form aufweist. Wie aus den Figuren 1 und 2 ersichtlich ist, nimmt die radiale Ausdehnung des Gelenkkopfs 20 nach ventral hin (also zur Vorderseite hin) leicht zu. Diese zusätzliche radiale Ausdehnung oder Materialzunahme des Gelenkkopfs 20 bleibt in Umfangsrichtung des Gelenkkopfs 20 im Wesentlichen auf die Vorderseite des Gelenkkopfs 20 beschränkt, so dass dort die in Figur 2 gezeigte Anschlagfläche 27 an den beiden äußeren Gelenkkopfabschnitten 23 entsteht. Eine in Relation zur Femurschaftachse geringfügige Dorsalverlagerung der Gelenkachse des Gelenkkopfs 20 verhindert, dass sich die einseitige Materialzunahme (bzw. radiale Ausdehnung) des Gelenkkopfs 20 nach ventral anatomisch ungünstig auswirkt und optimiert die Gelenkeinrichtung 100 gemäß den Erfordernissen.

Der Gelenkfuß 40 der in Figur 2 gezeigten Gelenkeinrichtung 100 umfasst zwei seitlich angeordnete Gelenkelemente 42. Diese seitlichen Gelenkelemente 42 sind jeweils an einem zentral angeordneten Gelenkelement 41 axial beabstandet voneinander angeordnet. Der axiale Abstand der beiden seitlichen Gelenkelemente 42 ist hierbei derart gewählt, dass die seitlichen Gelenkelemente 42 relativ zu den äußeren Gelenkkopfabschnitten 23a, 23b axial weiter außen liegen. Somit wird der Gelenkkopf 20 zwischen den beiden seitlichen Gelenkelementen 42 um die Gelenkachse drehbar gelagert, wie weiter unten noch näher beschrieben wird.

Die seitlichen Gelenkelemente 42 können mit Hilfe von Schrauben 42a am zentralen Gelenkelement 41 des Gelenkfußes 40 lösbar befestigt sein. Andere Befestigungsmittel sind ebenso denkbar. An den beiden seitlichen Gelenkelementen 42 können ferner Justiernasen 42b vorgesehen sein, welche dazu ausgebildet sind, in entsprechende Justiernuten (in der Figur 2 nicht sichtbar) am zentralen Gelenkelement 41 formschlüssig einzugreifen. Dadurch wird die korrekte Montage, insbesondere die korrekte Ausrichtung der beiden lateralen Gelenkelemente 42 bezüglich des zentralen Gelenkelements 41 sichergestellt. Ferner können die Justiernasen 42b als stabile Formschlusselemente ausgebildet sein, um Scherkräfte und Biegemomente von den Schrauben 42a abzuhalten, wodurch die mechanische Beständigkeit der Gelenkeinrichtung 100 weiter erhöht wird und damit der hohen Beanspruchung gerecht wird. Die Schrauben 42a sind demnach lediglich Axialkräften ausgesetzt.

Am zentralen Gelenkelement 41 ist ferner ein Anschlagelement 43 zwischen den beiden seitlichen Gelenkelementen 42 angeordnet. Es ist dazu ausgebildet, bei einer Streckbewegung mit der wenigstens einen Anschlagfläche 27 des Gelenkkopfs 20 in Anschlag zu kommen und somit eine Überstreckung des Gelenks zu verhindern. Mit anderen Worten wird mit Hilfe der wenigstens einen Anschlagfläche 27 am Gelenkkopf 20 und dem Anschlagelement 43 eine maximal mögliche Streckstellung der Gelenkeinrichtung 100 festgelegt.

Das zentrale Gelenkelement 41 kann an seiner von dem Anschlagelement 43 und den zwei seitlichen Gelenkelementen 42 abgewandten Seite mit einem (nicht gezeigten) tibialen Knochenschaftersatzelement verbunden sein. Der Gelenkfuß 40 weist einen Fixierungsfortsatz 47 auf, durch den eine Durchgangsöffnung 48 verläuft. Die Durchgangsöffnung 48 kann wiederum in einen im zentralen Gelenkkopfabschnitt 23z ausgebildeten Führungskanal 14 münden. Durch die Durchgangsöffnung 48 des Gelenkfußes 40 kann beispielsweise eine tibiale Schaftverankerung (in der Figur 1 nicht dargestellt), ein Werkzeug oder ein Distraktionsmarknagel zur Tibia hindurchgeführt werden. Insbesondere kann der Distraktionsmarknagel oder die tibiale Schaftverankerung mittels Befestigungsstiften am Fixierungsfortsatz 47 befestigt werden, indem die Befestigungsstifte durch in der Wandung des Fixierungsfortsatzes 47 vorgesehene Querbohrungen 49 geführt werden.

Die zwei seitlichen Gelenkelemente 42 bilden zusammen mit dem zentralen Gelenkelement 41 den gabelförmigen Gelenkfuß 40. Die beiden seitlichen Gelenkelemente 42 weisen jeweils eine axiale Innenfläche 44 auf, an denen jeweils ein ringförmiger Vorsprung 46 (im Folgenden auch Innenring 46 genannt) ausgebildet ist. Der jeweilige ringförmige Vorsprung 46 weist eine kreisförmige Außenfläche 45 auf, welche die zweite Gleitfläche 45 (Gelenkfläche 45) der Gelenkeinrichtung 100 bildet.

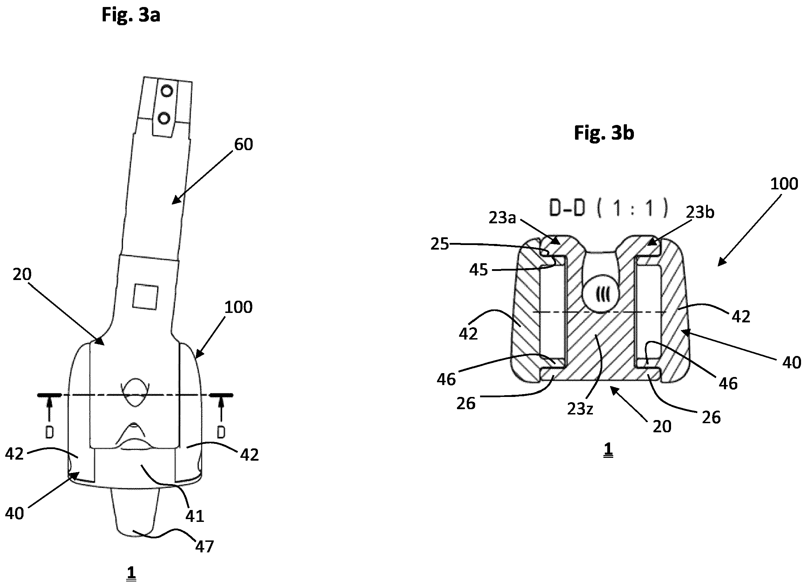

In Zusammenhang mit den Figuren 3a und 3b wird das Zusammenwirken der jeweiligen ersten und zweiten Gleitflächen (Gelenkflächen) 25 und 45 der Gelenkeinrichtung 100 weiter beschrieben. Figur 3a zeigt eine Draufsicht auf die Prothese 1 mit der Gelenkeinrichtung 100. Figur 3b zeigt eine Schnittansicht entlang der Schnittebene D-D. Im montierten Zustand greifen die jeweiligen Innenringe 46 der beiden seitlichen Gelenkelemente 42 in die jeweiligen Außenringe 26 des Gelenkkopfs 20 ein. Dadurch kommen die kreisförmig gekrümmten zweiten Gleitflächen 45 der seitlichen Gelenkelemente 42 mit den kreisförmig gekrümmten ersten Gleitflächen 25 des Gelenkkopfs 20 in Gleiteingriff. Somit kann der Gelenkfuß 40 relativ zum Gelenkkopf 20 um die Gelenkachse gedreht (rotiert) werden und somit eine Beuge- oder Streckbewegung realisieren. Zur Verbesserung der Gleitfähigkeit können die jeweiligen Gleitflächen 25, 45 jeweils mit einer biokompatiblen Beschichtung versehen sein oder aus einer verschleißresistenten und reibungsmindernden Materialkomponente, wie sie aus dem Stand der Technik bekannt sind, gefertigt sein.

Zurück zur Figur 2. Die lösbare Montage der beiden seitlichen Gelenkelemente 42 am zentralen Gelenkelement 41 (diese erfolgt mit Hilfe der oben beschriebenen Schrauben 42a und Justiernasen 42b) ermöglicht eine flexible Montage der Gelenkeinrichtung 100 (und somit der Prothese 1) während des operativen Eingriffs, ohne dass die Stabilität dadurch beeinträchtigt wird. So kann die Montage der Gelenkeinrichtung 100 sowohl von medial als auch von lateral erfolgen, abhängig vom operativen Zugang. In beiden Montagevarianten kann die Prothese 1 beim operativen Eingriff zunächst vormontiert werden, wobei bei einem Einsetzen der Prothese 1 von der medialen/lateralen Seite zunächst das von der medialen/lateralen Seite abgewandte laterale/mediale Gelenkelement 42 am zentralen Gelenkelement 41 vormontiert wird. Nach dem Einsetzen der Prothese 1 kann das der medialen/ lateralen Seite zugewandte Gelenkelement 42 am zentralen Gelenkelement 41 befestigt werden, wodurch die Gelenkeinrichtung 100 geschlossen wird.

Durch die hier beschriebene Ausgestaltung der Gelenkeinrichtung 100, bei der die gelenkige Lagerung zwischen dem Gelenkkopf 20 einerseits und dem gabelförmigen Gelenkfuß 40 andererseits axial so weit wie anatomisch möglich nach außen verlagert ist, wird die Stabilität der Gelenkeinrichtung 100 insgesamt verbessert. Dem zentralen Gelenkkopfabschnitt 23z, in welchem die Führungskanäle 12, 14 zur Durchführung von Werkzeug, Distraktionsmarknagel oder einer femoralen oder tibialen Schaftverankerung vorgesehen sind, kommt keine gelenkstabilisierende Funktion zu.

Die Gelenkeinrichtung 100 ist insgesamt dazu ausgebildet, eine Streck- und/oder Beugebewegung entsprechend einem natürlichen Kniegelenk durchzuführen. Dies geschieht durch Drehung/Drehbewegung des Gelenkfußes 40 relativ zum Gelenkkopf 20 um die Gelenkachse. Bevorzugt kann die Gelenkeinrichtung 100, ausgehend von einer Streckstellung, einen maximalen Beugungswinkel von 120° realisieren. Die Streckstellung wird bei der Drehbewegung dann erreicht, wenn das zentrale Gelenkelement 41 des Gelenkfußes 40 an der wenigstens einen Anschlagfläche 27 des Gelenkkopfs 20 in Anschlag kommt.

Die Ventralseite des Gelenkkopfes 20 kann ferner derart ausgebildet sein, dass sie eine Gleitfläche für die Kniescheibe bildet. Die Außenöffnungen 12a und 14a der Führungskanäle 12 und 14 liegen dabei in einem Bereich des Gelenkkopfes 20, der nicht mit der Unterfläche der Kniescheibe in Berührung kommt, so dass die Bewegung der Kniescheibe nicht beeinträchtigt ist.

Die Führungskanäle 12 und 14 (siehe Figur 2) sind in Form von Durchgangsbohrungen im zentralen Gelenkkopfabschnitt 23z realisiert mit zur Ventralseite hin ausgebildeten Außenöffnungen 12a, 14a. Die Führungskanäle 12 und 14 (Durchgangsbohrungen) sind im zentralen Gelenkkopfabschnitt 23z derart angeordnet, dass jeweils ein Führungskanal 12, 14 mit einer Durchgangsöffnung des femoralen Knochenschaftersatzelements 60 bzw. mit der Durchgangsöffnung 48 des Gelenkfußes 40 und ggf. des tibialen Knochenschaftersatzelements in Ausrichtung kommt (in Figur 2 nicht dargestellt), wenn die Prothese 1 bzw. ihre Gelenkeinrichtung 100 eine einer Kniebeugung entsprechende vorgegebene Beugestellung (vorgegebenen Beugungswinkel) realisiert.

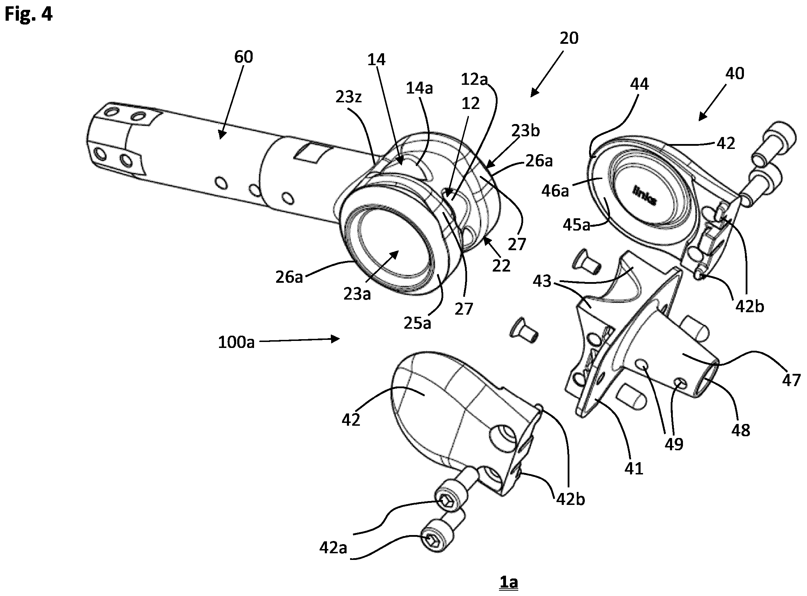

In Zusammenhang mit der Figur 4 wird eine weitere Variante einer erfindungsgemäßen Prothese 1a mit einer Gelenkeinrichtung 100a zum Ersatz des menschlichen Kniegelenks beschrieben.

Figur 4 zeigt eine Explosionsdarstellung der Prothese 1a. Sie unterscheidet sich von der Prothese 1 gemäß den Figuren 1, 2, 3a und 3b in der Ausbildung der ersten und zweiten Gleitflächen der Gelenkeinrichtung 100a und somit in der gelenkigen Kopplung zwischen dem Gelenkkopf 20 und dem Gelenkfuß 40. Alle anderen Komponenten der Prothese 1a bzw. der Gelenkeinrichtung 100a sind strukturell und funktionell identisch zur Prothese 1 bzw. Gelenkeinrichtung 100 der Figuren 1, 2, 3a und 3b, so dass auf die obige Beschreibung verwiesen wird, um unnötige Wiederholungen zu vermeiden. Im Folgenden werden lediglich die Unterschiede zur Gelenkeinrichtung 100 der Prothese 1 gemäß den Figuren 1, 2, 3a und 3b weiter beschrieben.

Im Unterschied zur Ausführungsform in den Figuren 1, 2, 3a und 3b weist der Gelenkkopf 20 an seinen gegenüberliegenden axialen Enden jeweils einen ringförmigen Vorsprung 26a mit einer konvex gewölbten, sphärischen Oberfläche 25a auf, die einem Abschnitt (Segment) einer Kugeloberfläche entspricht. Die konvex gewölbte, sphärische Oberfläche 25a fungiert als erste Gleitfläche 25a des Gelenkkopfs 20. Ferner weisen die zwei axial beabstandet angeordneten seitlichen Gelenkelemente 42 des gabelförmigen Gelenkfußes 40 an ihren axialen Innenflächen 44 jeweils eine zu den ringförmigen Vorsprüngen 26a korrespondierende ringförmige Nut 46a auf. Die jeweilige ringförmige Nut 46a weist eine konkav gewölbte, sphärische Oberfläche 45a auf, die als zweite Gleitfläche 45a fungiert und einem Abschnitt (Segment) einer Kugeloberfläche entspricht.

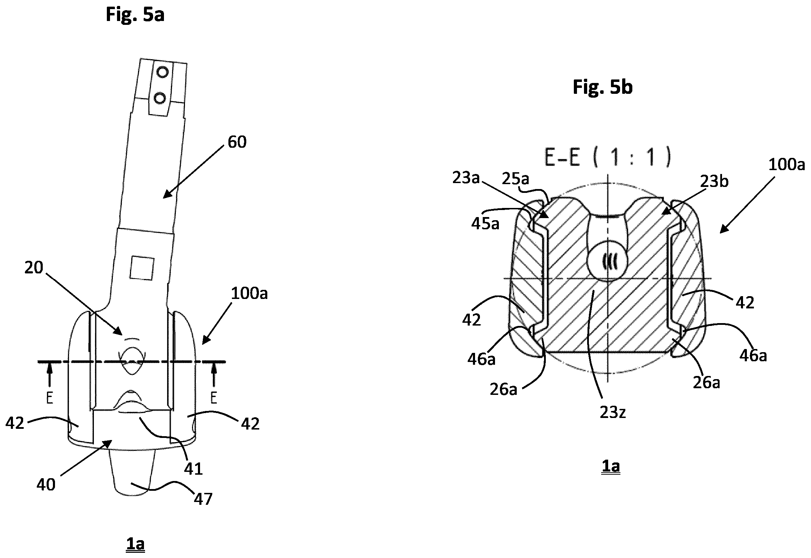

In Zusammenhang mit den Figuren 5a und 5b wird das Zusammenwirken der jeweiligen ersten und zweiten Gleitflächen (Gelenkflächen) 25a und 45a der Gelenkeinrichtung 100a weiter beschrieben. Figur 5a zeigt eine Draufsicht auf die Prothese 1a mit der Gelenkeinrichtung 100a. Figur 5b zeigt eine Schnittansicht entlang der Schnittebene E-E. Im montierten Zustand greifen die an den axialen Enden des Gelenkkopfs 20 angeordneten ringförmigen Vorsprünge 26a in die an den axialen Innenflächen 44 der seitlichen Gelenkelemente 42 angeordneten und den ringförmigen Vorsprüngen 26a gegenüberliegenden ringförmigen Nuten 46a ein. Dadurch kommen die sphärisch konvex ausgebildeten ersten Gleitflächen 25a des Gelenkkopfs 20 mit den jeweiligen sphärisch konkav ausgebildeten zweiten Gleitflächen 45a des Gelenkfußes 40 in gleitende Berührung (siehe Figur 5b).

Durch diese Ausgestaltung wird nicht nur eine Drehbewegung um die Gelenkachse möglich, sondern zusätzlich auch eine begrenzte Rotation (um einige Grad) um eine senkrecht zur Gelenkachse angeordnete und durch den Gelenkfuß 40 verlaufenden Rotationsachse. Diese zusätzliche Rotation ist insbesondere dann möglich, wenn die Gelenkeinrichtung 100a sich in einer Beugestellung befindet. Befindet sich hingegen die Gelenkeinrichtung 100a in voller Streckstellung, bei der das Anschlagelement 43 des Gelenkfußes 40 mit der wenigstens einen Anschlagfläche 27 des Gelenkkopfs 20 im Anschlag ist, wird diese zusätzliche Rotation durch das Zusammenwirken von Anschlagelement 43 mit der wenigstens einen Anschlagfläche 27 unterbunden. Durch die hier beschriebene zusätzliche leichte Rotation senkrecht zur Gelenkachse in Beugestellung und die Aufhebung der Rotation in Streckstellung kann die Beweglichkeit eines Kniegelenks noch physiologischer nachgebildet werden, indem bei abgewinkelter Kniestellung eine begrenzte rotatorische Bewegung um die Tibiaachse möglich ist.

### Bezugszeichenliste

- 1, 1a: Prothese
- 12: erste Durchgangsbohrung/erster Führungskanal
- 12a: Außenöffnung
- 14: zweite Durchgangsbohrung/zweiter Führungskanal
- 14a: Außenöffnung
- 20: Gelenkkopf
- 22: Walzenkörper
- 23a, 23b: äußere Gelenkkopfabschnitte
- 23z: zentraler Gelenkkopfabschnitt
- 25, 25a: erste Gleitfläche(n)
- 26: Außenring
- 26a: ringförmiger Vorsprung
- 27: Anschlagfläche(n)
- 40: Gelenkfuß
- 41: zentrales Gelenkelement
- 42: seitliche(s) Gelenkelement(e)
- 42a: Schrauben
- 42b: Justiernasen
- 43: Anschlagelement
- 44: axiale Innenfläche(n) des(der) seitlichen Gelenkelements(e)
- 45, 45a: zweite Gleitfläche(n)
- 46: Innenring
- 46a: ringförmige Nut
- 47: Fixierungsfortsatz
- 48: Durchgangsöffnung Gelenkfuß
- 49: Querbohrung
- 60: femorales Knochenschaftersatzelement
- 100, 100a: Gelenkeinrichtung

## Patentansprüche

1. Implantierbare Prothese (1, 1a) zum Ersatz eines menschlichen Kniegelenks, umfassend eine Gelenkeinrichtung (100, 100a) mit einem femoralen Gelenkkopf (20) und einen mit dem Gelenkkopf (20) zusammenwirkenden tibialen Gelenkfuß (40), die zur wahlweisen Realisierung wenigstens einer Gelenkbewegung in Form einer Beuge- und/oder Streckbewegung vorgesehen sind,
wobei der Gelenkkopf (20) zwei erste Gleitflächen (25, 25a) aufweist, die an gegenüberliegenden axialen Enden (24) des Gelenkkopfs (20) angeordnet sind und durch einen zentralen Gelenkkopfabschnitt (23z) räumlich voneinander getrennt sind,
wobei der Gelenkfuß (40) zwei zweite Gleitflächen (45, 45a) aufweist, die an seitlich angeordneten Gelenkelementen (42) des Gelenkfußes (40) axial gegenüberliegend ausgebildet sind und mit den jeweiligen ersten Gleitflächen (25, 25a) des Gelenkkopfs (20) in Gleiteingriff stehen, wobei die zwei ersten Gleitflächen (25, 25a) und die zwei zweiten Gleitflächen (45, 45a) zur Realisierung wenigstens einer Beuge- und/oder Streckbewegung ausgebildet sind, und
wobei der zentrale Gelenkkopfabschnitt (23z) baulich für die Gelenkfunktion entbehrlich ist, **dadurch gekennzeichnet, dass**
der zentrale Gelenkkopfabschnitt (23z)
zwei Führungskanäle (12, 14) aufweist, die jeweils zur Durchführung eines Werkzeuges, eines Distraktionsmarknagels oder einer femoralen oder tibialen Schaftverankerung vorgesehen sind.

2. Implantierbare Prothese (1, 1a) nach Anspruch 1, wobei die Prothese (1, 1a) ein femorales Knochenschaftersatzelement (60) und/oder ein tibiales Knochenschaftersatzelement umfasst, wobei der Gelenkkopf (20) mit dem distalen Ende des femoralen Knochenschaftersatzelements (60) verbunden ist oder eine Einheit bildet, wobei der Gelenkfuß (40) mit dem proximalen Ende des tibialen Knochenschaftersatzelements verbunden ist oder eine Einheit bildet.

3. Implantierbare Prothese (1, 1a) nach Anspruch 1 oder 2, wobei der Gelenkkopf (20) zwei äußere Gelenkkopfabschnitte (23a, 23b) aufweist, welche in axialer Richtung durch den zentralen Gelenkkopfabschnitt (23z) räumlich voneinander getrennt sind, wobei jeweils eine der zwei ersten Gleitflächen (25, 25a) am axialen äußeren Ende eines jeden äußeren Kopfabschnittes (23a, 23b) ausgebildet ist.

4. Implantierbare Prothese (1, 1a) nach Anspruch 1 oder 2, wobei der Gelenkkopf (20) im Wesentlichen walzenförmig ausgebildet ist.

5. Implantierbare Prothese (1, 1a) nach einem der Ansprüche 1 bis 4, wobei der Gelenkfuß (40) gabelförmig ausgebildet ist mit zwei zur beweglichen Aufnahme des Gelenkkopfs (20) ausgebildeten seitlichen Gelenkelementen (42), die am Gelenkfuß (40) axial voneinander beabstandet angeordnet sind.

6. Implantierbare Prothese (1, 1a) nach Anspruch 5, wobei der Gelenkfuß (40) ein zentrales Gelenkelement (41) aufweist, wobei die seitlichen Gelenkelemente (42) am zentralen Gelenkelement (41) lösbar befestigbar sind, wobei optional die zwei seitlichen Gelenkelemente (42) Justiernasen (42b) aufweisen, die dazu ausgebildet sind, in entsprechende Justiernuten am zentralen Gelenkelement (41) formschlüssig einzugreifen.

7. Implantierbare Prothese (1, 1a) nach Anspruch 5 oder 6, wobei die zwei zweiten Gleitflächen (45, 45a) an den Innenseiten der zwei seitlich angeordneten Gelenkelemente (42) ausgebildet sind.

8. Implantierbare Prothese (1) nach einem der Ansprüche 1 bis 7, wobei die Gelenkeinrichtung (100, 100a) dazu ausgebildet ist, ausgehend von einer Streckstellung eine Beugebewegung von bis zu 120° zu realisieren.

9. Implantierbare Prothese (1, 1a) nach einem der Ansprüche 1 bis 8, wobei die Gelenkeinrichtung (100, 100a) ferner einen Streckanschlag umfasst, welcher zur Begrenzung der Streckbewegung vorgesehen ist, wobei der Streckanschlag durch wenigstens eine am Gelenkkopf (20) ausgebildete Anschlagfläche (27) realisiert ist.

10. Implantierbare Prothese (1, 1a) nach einem der Ansprüche 1 bis 9, wobei die zwei ersten Gleitflächen (25, 25a) und die zwei zweiten Gleitflächen (45, 45a) ausgebildet sind, um zusätzlich zu der Beuge- und/oder Streckbewegung um die Gelenkachse eine dazu senkrechte, begrenzte Rotationsbewegung zu ermöglichen, wenn die Gelenkeinrichtung (100, 100a) sich in einer Beugestellung befindet.

11. Implantierbare Prothese (1, 1a) nach einem der Ansprüche 1 bis 10, wobei die zwei ersten Gleitflächen (25a) und die zwei zweiten Gleitflächen (45a) jeweils sphärisch gekrümmte Gleitflächen sind.

12. Implantierbare Prothese (1, 1a) nach einem der Ansprüche 1 bis 9, wobei die zwei ersten Gleitflächen (25) und die zwei zweiten Gleitflächen (45) jeweils kreisförmig gekrümmte Gleitflächen sind.

13. Implantierbare Prothese (1, 1a) nach einem der Ansprüche 1 bis 12, wobei die zwei ersten Gleitflächen (25, 25a) und/oder die zwei zweiten Gleitflächen (45, 45a) eine biokompatible Beschichtung aufweisen oder aus einer zusätzlichen Materialkomponente zur Reibungsverminderung bestehen.

14. Implantierbare Prothese (1, 1a) nach einem der Ansprüche 1 bis 13, wobei die Gelenkeinrichtung (100, 100a) ferner eine Gleitfläche für eine Kniescheibe aufweist.

15. Implantierbare Prothese (1, 1a) nach einem der Ansprüche 1 bis 14, wobei die zwei Führungskanäle (12, 14) im zentralen Gelenkkopfabschnitt (23z) derart ausgerichtet angeordnet sind, dass ein Werkzeug, ein Distraktionsmarknagel oder eine femorale Schaftverankerung über einen ersten Führungskanal (12) der zwei Führungskanäle (12, 14) in eine angrenzende Durchgangsöffnung eines femoralen Knochenschaftersatzelements (60) und weiter in den Femur eingeführt werden kann, und ein Werkzeug, ein Distraktionsmarknagel oder eine tibiale Schaftverankerung über einen zweiten Führungskanal (14) der zwei Führungskanäle (12, 14) in eine angrenzende Durchgangsöffnung (48) des tibialen Gelenkfußes (40) und weiter in die Tibia eingeführt werden kann, wenn sich die Gelenkeinrichtung (100, 100a) in einem vorgegebenen Beugungswinkel befindet.

## Claims

1. An implantable prosthesis (1, 1a) for replacement of a human knee joint, comprising a joint device (100, 100a) with a femoral joint head (20) and a tibial joint base (40) interacting with the joint head (20), which are provided for selectively realizing at least one joint movement in the form of a bending and/or extension movement,
wherein the joint head (20) has two first sliding surfaces (25, 25a) which are arranged at opposite axial ends (24) of the joint head (20) and are spatially separated from each other by a central joint head section (23z),
wherein the joint base (40) has two second sliding surfaces (45, 45a) which are formed axially opposite each other on laterally arranged joint elements (42) of the joint base (40) and are in sliding engagement with the respective first sliding surfaces (25, 25a) of the joint head (20), wherein the two first sliding surfaces (25, 25a) and the two second sliding surfaces (45, 45a) are formed to realize at least one bending and/or extension movement, and
wherein the central joint head section (23z) is structurally dispensable for the joint function,
**characterised in that** the central joint head section (23z) has two guide channels (12, 14), each of which is provided for passing a tool, a distraction intramedullary nail or a femoral or tibial shaft anchor.

2. The implantable prosthesis (1, 1a) according to claim 1, wherein the prosthesis (1, 1a) comprises a femoral bone shaft replacement element (60) and/or a tibial bone shaft replacement element, wherein the joint head (20) is connected to the distal end of the femoral bone shaft replacement element (60) or forms a unit therewith, wherein the joint base (40) is connected to the proximal end of the tibial bone shaft replacement element or forms a unit therewith.

3. The implantable prosthesis (1, 1a) according to claim 1 or 2, wherein the joint head (20) comprises two outer joint head sections (23a, 23b) which are spatially separated from each other in the axial direction by the central joint head section (23z), wherein one of the two first sliding surfaces (25, 25a) is formed at the axial outer end of each outer head section (23a, 23b).

4. The implantable prosthesis (1, 1a) according to claim 1 or 2, wherein the joint head (20) is essentially cylindrical in shape.

5. The implantable prosthesis (1, 1a) according to one of claims 1 to 4, wherein the joint base (40) is fork-shaped with two lateral joint elements (42) formed to movably receive the joint head (20), which are arranged on the joint base (40) at an axial distance from each other.

6. The implantable prosthesis (1, 1a) according to claim 5, wherein the joint base (40) has a a central joint element (41), wherein the lateral joint elements (42) are detachably fastenable to the central joint element (41), wherein, optionally, the two lateral joint elements (42) have adjusting lugs (42b) which are formed to engage positively in corresponding adjusting grooves on the central joint element (41).

7. The implantable prosthesis (1, 1a) according to claim 5 or 6, wherein the two second sliding surfaces (45, 45a) are formed on the inner sides of the two laterally arranged joint elements (42).

8. The implantable prosthesis (1) according to one of claims 1 to 7, wherein the joint device (100, 100a) is formed to realize a bending movement of up to 120° starting from an extended position.

9. The implantable prosthesis (1, 1a) according to one of claims 1 to 8, wherein the joint device (100, 100a) further comprises an extension stop, which is provided to limit the extension movement, wherein the extension stop is realized by at least one stop surface (27) formed on the joint head (20).

10. The implantable prosthesis (1, 1a) according to one of claims 1 to 9, wherein the two first sliding surfaces (25, 25a) and the two second sliding surfaces (45, 45a) are formed to enable, in addition to the bending and/or extension movement about the joint axis, a limited rotational movement perpendicular thereto when the joint device (100, 100a) is in a bent position.

11. The implantable prosthesis (1, 1a) according to one of claims 1 to 10, wherein the two first sliding surfaces (25a) and the two second sliding surfaces (45a) are each spherically curved sliding surfaces.

12. The implantable prosthesis (1, 1a) according to one of claims 1 to 9, wherein the two first sliding surfaces (25) and the two second sliding surfaces (45) are each circularly curved sliding surfaces.

13. The implantable prosthesis (1, 1a) according to one of claims 1 to 12, wherein the two first sliding surfaces (25, 25a) and/or the two second sliding surfaces (45, 45a) have a biocompatible coating or consist of an additional material component for reducing friction.

14. The implantable prosthesis (1, 1a) according to one of claims 1 to 13, wherein the joint device (100, 100a) further comprises a sliding surface for a kneecap.

15. The implantable prosthesis (1, 1a) according to one of claims 1 to 14, wherein the two guide channels (12, 14) are arranged in an aligned manner in the central joint head section (23z) in such a way that a tool, a distraction intramedullary nail or a femoral shaft anchor can be inserted via a first guide channel (12) of the two guide channels (12, 14) into an adjacent through-hole of a femoral bone shaft replacement element (60) and further into the femur, and a tool, a distraction intramedullary nail or a tibial shaft anchor can be inserted via a second guide channel (14) of the two guide channels (12, 14) into an adjacent through-hole (48) of the tibial joint base (40) and further into the tibia when the joint device (100, 100a) is at a predetermined bending angle.

## Revendications

1. Prothèse implantable (1, 1a) destinée au remplacement d'une articulation du genou humain, comprenant un dispositif articulaire (100, 100a) comportant une tête articulaire fémorale (20) et un pied articulaire tibial (40) coopérant avec la tête articulaire (20), qui sont prévus pour permettre, sélectivement la réalisation d'au moins un mouvement articulaire sous la forme d'un mouvement de flexion et/ou d'extension,
dans laquelle la tête articulaire (20) présente deux premières surfaces de glissement (25, 25a) qui sont disposées aux extrémités axiales opposées (24) de la tête articulaire (20) et qui sont séparées spatialement l'une de l'autre par une partie centrale (23z) de la tête articulaire,
dans laquelle le pied articulaire (40) présente deux deuxièmes surfaces de glissement (45, 45a) qui sont formées sur des éléments articulaires (42) disposés latéralement sur le pied d'articulation (40), de manière axialement opposée, et qui sont en contact de glissement avec les premières surfaces de glissement respectives (25, 25a) de la tête articulaire (20), les deux premières surfaces de glissement (25, 25a) et les deux deuxièmes surfaces de glissement (45, 45a) étant configurées pour permettre au moins un mouvement de flexion et/ou d'extension, et
dans laquelle la partie centrale (23z) de la tête articulaire est structurellement superflue pour la fonction articulaire,
**caractérisée en ce que** la partie centrale (23z) de la tête articulaire comporte deux canaux de guidage (12, 14) qui sont respectivement destinés au passage d'un outil, d'un clou médullaire de distraction ou d'un ancrage de tige fémoral ou tibial.

2. Prothèse implantable (1, 1a) selon la revendication 1, dans laquelle la prothèse (1, 1a) comprend un élément de remplacement de la tige osseuse fémorale (60) et/ou un élément de remplacement de la tige osseuse tibiale, dans laquelle la tête articulaire (20) est reliée à l'extrémité distale de l'élément de remplacement de la tige osseuse fémorale (60) ou forme une seule unité avec celle-ci, le pied (40) étant relié à l'extrémité proximale de l'élément de remplacement de la tige osseuse tibiale ou formant une seule unité avec celle-ci.

3. Prothèse implantable (1, 1a) selon la revendication 1 ou 2, dans laquelle la tête articulaire (20) comporte deux parties de tête articulaire extérieures (23a, 23b) qui sont spatialement séparées l'une de l'autre dans la direction axiale par la partie centrale (23z) de la tête articulaire, l'une des deux premières surfaces de glissement (25, 25a) étant formée à l'extrémité axiale extérieure de chacune des parties de tête extérieures (23a, 23b).

4. Prothèse implantable (1, 1a) selon la revendication 1 ou 2, dans laquelle la tête articulaire (20) est de forme essentiellement cylindrique.

5. Prothèse implantable (1, 1a) selon l'une des revendications 1 à 4, dans laquelle le pied articulaire (40) est de forme fourchue et comporte deux éléments articulaires latéraux (42) configurés pour recevoir de manière mobile la tête articulaire (20), lesquels sont disposés sur le pied articulaire (40) à distance axiale l'un de l'autre.

6. Prothèse implantable (1, 1a) selon la revendication 5, dans laquelle le pied articulaire (40) comporte un élément articulaire central (41), les éléments articulaires latéraux (42) pouvant être fixés de manière amovible à l'élément articulaire central (41), dans laquelle les deux éléments articulaires latéraux (42) comportent des ergots d'ajustement (42b) configurés pour s'engager par complémentarité de forme dans des rainures d'ajustement correspondantes de l'élément d'articulation central (41).

7. Prothèse implantable (1, 1a) selon la revendication 5 ou 6, dans laquelle les deux secondes surfaces de glissement (45, 45a) sont formées sur les faces intérieures des deux éléments d'articulation (42) disposés latéralement.

8. Prothèse implantable (1) selon l'une des revendications 1 à 7, dans laquelle le dispositif articulaire (100, 100a) est configuré pour réaliser, à partir d'une position d'extension, un mouvement de flexion allant jusqu'à 120°.

9. Prothèse implantable (1, 1a) selon l'une des revendications 1 à 8, dans laquelle le dispositif articulaire (100, 100a) comprend en outre une butée d'extension destinée à limiter le mouvement d'extension, ladite butée d'extension étant réalisée par au moins une surface de butée (27) formée sur la tête articulaire (20).

10. Prothèse implantable (1, 1a) selon l'une des revendications 1 à 9, dans laquelle les deux premières surfaces de glissement (25, 25a) et les deux secondes surfaces de glissement (45, 45a) sont configurées pour permettre, en plus du mouvement de flexion et/ou d'extension autour de l'axe articulaire, un mouvement de rotation limité et perpendiculaire à celui-ci lorsque le dispositif articulaire (100, 100a) se trouve en position de flexion.

11. Prothèse implantable (1, 1a) selon l'une des revendications 1 à 10, dans laquelle les deux premières surfaces de glissement (25a) et les deux secondes surfaces de glissement (45a) sont chacune des surfaces de glissement à courbure sphérique.

12. Prothèse implantable (1, 1a) selon l'une des revendications 1 à 9, dans laquelle les deux premières surfaces de glissement (25) et les deux secondes surfaces de glissement (45) sont chacune des surfaces de glissement à courbure circulaire.

13. Prothèse implantable (1, 1a) selon l'une des revendications 1 à 12, dans laquelle les deux premières surfaces de glissement (25, 25a) et/ou les deux secondes surfaces de glissement (45, 45a) présentent un revêtement biocompatible ou sont constituées d'un composant matériel supplémentaire destiné à réduire le frottement.

14. Prothèse implantable (1, 1a) selon l'une des revendications 1 à 13, dans laquelle le dispositif articulaire (100, 100a) comporte en outre une surface de glissement pour une rotule.

15. Prothèse implantable (1, 1a) selon l'une des revendications 1 à 14, dans laquelle les deux canaux de guidage (12, 14) sont disposés dans la partie centrale (23z) de la tête articulaire de manière alignée, de sorte qu'un outil, un clou médullaire de distraction ou un ancrage fémoral puisse être introduit via un premier canal de guidage (12) des deux canaux de guidage (12, 14) dans une ouverture de passage adjacente d'un élément de remplacement de la tige osseuse fémorale (60), puis dans le fémur, et qu'un outil, un clou médullaire de distraction ou un ancrage de tige tibiale puisse être introduit via un deuxième canal de guidage (14) des deux canaux de guidage (12, 14) dans une ouverture de passage adjacente (48) du pied articulaire tibial (40), puis dans le tibia, lorsque le dispositif articulaire (100, 100a) se trouve à un angle de flexion prédéfini.
